# FASCICULE DE BREVET EUROPEEN

(11) **EP 0 951 473 B1**
(45) Date de publication et mention de la délivrance du brevet: **05.06.2002**
(21) Numéro de dépôt: 97952997.1
(22) Date de dépôt: 23.12.1997
(51) Int. Cl.: C07K 7/64, A61K 38/13

(54) **DERIVE DE CYCLOSPORINE, SA PREPARATION ET LES COMPOSITIONS PHARMACEUTIQUES QUI LE CONTIENNENT**
CYCLOSPORINDERIVAT, DESSEN HERSTELLUNG UND PHARMAZEUTISCHE ZUSAMMENSETZUNG
CYCLOSPORIN DERIVATIVE, ITS PREPARATION AND PHARMACEUTICAL COMPOSITIONS CONTAINING SAME

(30) Priorité: 24.12.1996 FR 9615954
(43) Date de publication de la demande: 27.10.1999
(73) Titulaire: Aventis Pharma S.A., 92160 Antony (FR)
(72) Inventeur: BARRIERE, Jean-Claude, F-91400 Bures sur Yvette (FR); BASHIARDES, Georges, F-94320 Thiais (FR); CARRY, Jean-Christophe, F-92190 Meudon (FR); EVERS, Michel, F-94510 La Queue en Brie (FR); FILOCHE, Bruno, F-94000 Créteil (FR); MIGNANI, Serge, F-92290 Châtenay Malabry (FR)
(74) Mandataire: Becker, Philippe
(86) Numéro de dépôt international: FR9702404
(87) Numéro de publication internationale: WO9828328

(56) Documents cités:
- EP-A- 0 194 972
- EP-A- 0 484 281
- WO-A-97/04005
- US-A- 4 814 323
- BILLICH A ET AL: "MODE OF ACTION OF SDZ NIM 811, A NONIMMUNOSUPPRESSIVE CYCLOSPORIN A ANALOG WITH ACTIVITY AGAINST HUMAN IMMUNODEFICIENCY VIRUS (HIV) TYPE 1: INTERFERENCE WITH HIV PROTEIN-CYCLOPHILIN A INTERACTIONS" JOURNAL OF VIROLOGY, vol. 69, no. 4, avril 1995, pages 2451-2461, XP002022423

## Description

La présente invention concerne un dérivé de cyclosporine de formule : 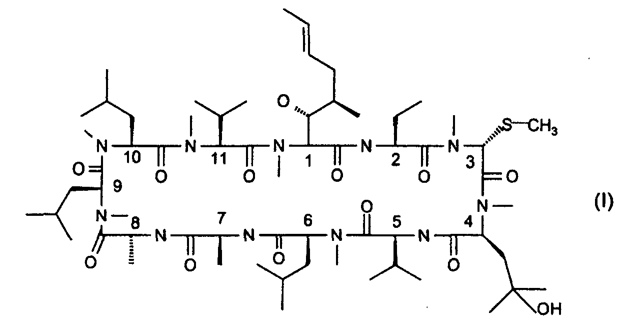 sa préparation et les compositions pharmaceutiques qui le contiennent.

Ce dérivé est utile pour le traitement et/ou la prophylaxie des infections à rétrovirus, et plus particulièrement du SIDA (syndrome d'immuno-déficience acquise) et de syndromes associés [ARC (AIDS related complex)]. Il présente l'avantage d'être très faiblement immunosuppresseur.

Des dérivés de cyclosporine modifiés en position -3 ont été précédemment décrits comme agents immunosuppresseurs, dans le brevet européen EP 194972.

Des dérivés de cyclosporine diversement modifiés et notamment le dérivé [4'-hydroxy-MeLeu]⁴ cyclosporine, ont été précédemment décrits dans le brevet européen EP 484281 et dans Eur. J. Immunol., 17, 1359 (1987). Ces dérivés sont utiles pour le traitement du SIDA et ne sont pas immunosuppresseurs.

Dans Bioorganic and Medicinal Chemistry Letters, 6(1), 23-26 (1996) a été décrit le dérivé de cyclosporine de formule : 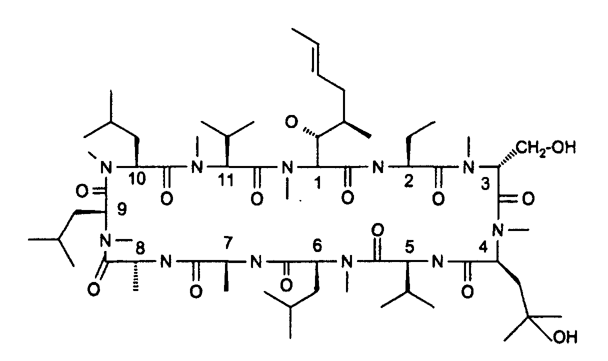 doué d'activité anti VIH-1.

Il a été trouvé maintenant que le dérivé de la cyclosporine de formule (I), est particulièrement intéressant du fait de son activité puissante et de son caractère très faiblement immunosuppresseur.

Selon la présente invention le produit de formule (I) peut être obtenu par action de disulfure de diméthyle sur une forme activée d'un dérivé [4'-hydroxy-MeLeu]⁴ cyclosporine de formule :

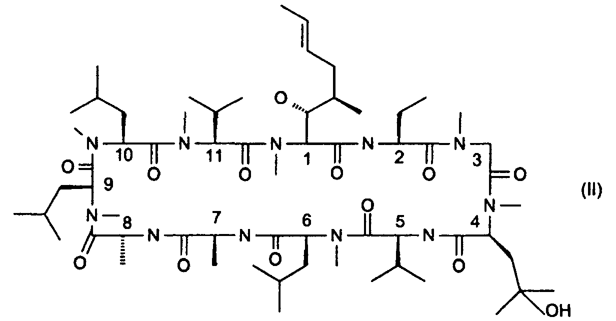

On entend par forme activée de la cyclosporine de formule générale (II), une forme activée sur la sarcosine en position 3. Cette forme activée est préparée de préférence in situ. L'activation s'effectue généralement sous atmosphère inerte, par traitement par un dérivé organométallique (lithien notamment, comme le n-butyllithium, le diisopropyl amidure de lithium ou un mélange par exemple).

L'addition du disulfure de diméthyle s'effectue avantageusement dans un solvant organique tel qu'un hydrocarbure (hexane par exemple) ou un éther (diéthyléther, tétrahydrofurane ou t.butylméthyléther par exemple) à une température comprise entre -78 et 0°C. De préférence on opère sous azote.

Le dérivé [4'-hydroxy-MeLeu]⁴ cyclosporine de formule (II) peut être préparé comme décrit dans la demande de brevet européen EP 484281.

Le nouveau dérivé de la cyclosporine de formule (I) peut être purifié le cas échéant par des méthodes physiques telles que la cristallisation ou la chromatographie.

Le nouveau dérivé de cyclosporine selon la présente invention est particulièrement utile pour la prophylaxie et le traitement des affections à rétrovirus et plus particulièrement du SIDA et de syndromes associés. Par prophylaxie on sous-entend notamment le traitement des sujets qui ont été exposés aux virus VIHs, en particulier les séropositifs asymptomatiques qui présentent le risque de développer la maladie dans les mois ou les années à venir après la primoinfection.

Le produit selon l'invention manifeste une activité anti-rétrovirus à des concentrations dépourvues d'effet cytotoxique ou cytostatique.

L'activité du produit de formule (I) a été mise en évidence dans les techniques décrites par Pauwells et Coll., J. Virol. Meth., 20, 309 (1988) et par O. Schwatz et Coll., AIDS Research and Human Retroviruses, 4(6), 441-48 (1988) et citées par J.F. Mayaux et al. Proc. Nat. Acad. Sci. USA, 91, 3564-68 (1994). Dans ces techniques la moyenne des concentrations actives est de 70 ou 50 nM (IC₅₀).

L'exemple suivant illustre la présente invention.

### Exemple RPR127933 (essai MOC 3752 lot MOC 3753)

La [(R)-méthylthio-Sar]³-[4'-hydroxy-MeLeu]⁴-cyclosporine A est préparée selon la méthode suivante:

A une solution de 25,2 cm³ de diisopropylamine (préalablement distillée sur hydrure de calcium) dans 330 cm³ de tétrahydrofuranne (préalablement distillé sur sodium) refroidie à une température voisine de -10°C et sous azote, on ajoute en 20 minutes , 111 cm³ d'une solution de n-butyllithium 1,6 M dans l'hexane en maintenant la température à 0°C. Le mélange est agité à 0°C pendant 20 minutes, puis refroidi à une température voisine de -78°C. La solution ainsi obtenue est transférée sous azote, par une canne de transfert, sur une solution de 14,45 g de [4'-hydroxy-MeLeu]⁴-cyclosporine A dans 290 cm³ de tétrahydrofuranne préalablement refroidie à une température voisine de -78°C en maintenant la température vers -68°C. Le mélange résultant est agité à une température voisine de -76°C pendant 20 minutes, puis 52 cm³ d'une solution de n-butyllithium 1,6 M dans. l'hexane sont ajoutés en 10 minutes. L'agitation est maintenue 30 minutes puis 22 cm³ de disulfure de diméthyle sont ajoutés en 10 minutes en maintenant la température vers -75°C. Le mélange est agité à une température voisine de -78°C pendant 2 heures puis à 0°C pendant 18 heures. On verse lentement sur le mélange réactionnel un mélange de 300 cm³ d'eau distillée et de 37 cm³ d'acide chlorhydrique aqueux à 36 %, puis le mélange est décanté. La phase aqueuse lavée par 200 cm³ d'éther diéthyligue. Les extraits organiques sont réunis, lavés avec 100 cm³ de solution saturée en chlorure de sodium, séchés sur sulfate de sodium, filtrés et concentrés sous pression réduite (2,7 kPa) à une température voisine de 40°C. Le solide obtenu est trituré dans 200 cm³ de pentane, filtré, lavé au pentane puis purifié par chromatographie flash sur colonne de silice (0,04-0,063 mm) en éluant avec un gradient (acétone 0 à 15 % - acétate d'éthyle 100-85 %, par volume de 500 cm³ par pas de 1 cm³) et en recueillant des fractions de 35 cm³. Les fractions contenant le produit attendu sont réunies et concentrées sous pression réduite (2,7 kPa) à une température voisine de 40°C pour donner un solide qui est concrété dans 20 cm³ de pentane. Après filtration, on obtient 2 g de [(R)-méthylthio-Sar]³-[4'-hydroxy-MeLeu]⁴-cyclosporine A sous la forme d'un solide blanc fondant vers 140°C.

Spectre de R.M.N. ¹ H (400 MHz, CDCl₃, δ en ppm) : 1,27 (d, J = 7 Hz, 3H : CH₃ 8β) ; 1,37 (d, J = 7,5 Hz, 3H : CH₃ 7β) ; 1,64 (d, J = 5 Hz, 3H : CH₃ en 1γ) ; de 1.65 à 1.80 et 2,41 (respectivement mt et dd, J = 15 et 6,5 Hz, 1H chacun : CH₂ 4β) ; 2,17 (s, 3H : SCH₃) ; 2,47 (mt, 1H : CH 5β) ; 2,71 - 3,13 - 3,18 - 3,27 - 3,46 et 3,52 (6 s, respectivement 6H - 3H - 3H - 3H - 3H et 3H : 7 NCH₃) ; 3,70 (d, J = 6,5 Hz, 1H : OH en 1β) ; 3,78 (mt, 1H : CH 1β) ; 4,56 (mt, 1H : CH 7α) ; 4,67 (t, J = 9 Hz, 1H : CH 5α) ; 4,86 (mt, 1H : CH 8α) ; 5,00 (dd, J = 9 et 6 Hz, 1H : CH α d'une leucine) ; de 5,05 à 5,15 (mt, 2H : CH 2α et CH α d'une leucine) ; 5,15 (d, J = 11 Hz, 1H : CH 11α) ; de 5,25 à 5,40 (mt, 2H : CH=CH) ; 5,45 (t, J = 6,5 Hz, 1H : CH 4α) ; 5,52 (d, J = 6 Hz, 1H : CH 1α) ; 5,72 (dd, J = 10,5 et 4 Hz, 1H : CH α d'une leucine) ; 5,75 (s, 1H : CH 3α) ; 7,16 (d, J = 8 Hz, 1H : CONH en 8) ; 7,52 (d, J = 9 Hz, 1H : CONH en 5) ; 7,65 (d, J = 7,5 Hz, 1H : CONH en 7) ; 7,94 (d, J = 9,5 Hz, 1H : CONH en 2).

La [4'-hydroxy-MeLeu]⁴-cyclosporine A peut être préparée selon la méthode décrite dans le brevet EP484281.

La présente invention concerne également les compositions pharmaceutiques contenant le produit de formule (I) le cas échéant sous forme de sel, à l'état pur ou sous forme d'une association avec un ou plusieurs diluants ou adjuvants compatibles et pharmaceutiquement acceptables, ou avec un autre agent antirétrovirus, éventuellement destiné au traitement du SIDA, un agent antiviral, immunomodulateur ou antimicrobien.

La composition selon l'invention est capable de maintenir en vie les cellules infectées par un rétrovirus comme par exemple le VIH et donc de réduire la progression vers le SIDA ou de diminuer sa gravité chez les sujets déjà infectés en réduisant la mortalité des cellules infectées. Les compositions peuvent être utilisées par voie orale, parentérale, rectale ou en aérosols.

Les compositions pharmaceutigues peuvent être utilisées à titre curatif ou à titre préventif chez des sujets présentant une immunodéficience et/ou infectés par un rétrovirus. Bien entendu, la constitution de ces compositions sera adaptée au cas particulier du tractus digestif des immunodéprimés.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon.

Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle.

Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène. dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, le médecin déterminera la posologie qu'il estime la plus appropriée en fonction d'un traitement préventif ou curatif, en fonction de l'âge, du poids, du degré de l'infection et des autres facteurs propres au sujet à traiter. Généralement, les doses comprises entre 5 et 30 mg/kg par voie orale pour un adulte.

Il a de plus été montré que le dérivé de cyclosporine de formule (I) manifeste un effet de synergie lorsqu'il est associé à d'autres agents anti-viraux actifs sur les rétrovirus. La présente invention concerne également les associations synergisantes qui contiennent le dérivé de la cyclosporine de formule (I) et un principe actif connu pour son activité sur les rétrovirus.

Les agents connus pour leur activité sur les rétrovirus qui peuvent être associés, sont choisis parmi des agents compatibles et inertes vis à vis du dérivé de cyclosporine de formule générale (I), aussi bien dans la catégorie des traitements pharmacologiques que dans la catégorie des traitements alternatifs tels que la thérapie génique et cellulaire ou antisens. A titre non limitatif ces agents constituant les differentes classes thérapeutiques sont choisis par exemple parmi des inhibiteurs nucléosidiques (NRTI) et non nucléosidiques (NNRTI) de la reverse transcriptase [zidovudine (AZT), didanosine (DDI), didéoxycytidine (DDC), d4T, ribavirine, 3TC, névirapine ...], parmi des inhibiteurs de la protéase (comma par exemple le Saquinavir, le Ritonavir, l'Indinavir et le Nelfinavir], des inhibiteurs de l'intégrase [comme le AR177], parmi les inhibiteurs de gene thérapie ciblant les protéines régulatrices de la réplication des VIHs tels que les inhibiteurs de la protéine rev [comme par exemple le Rev M10], ou les inhibiteurs de la nucléocapside [comme par exemple les DIBAs], parmi les inhibiteurs ciblant les transcripts RNA messager specifiques de tous les VIHs comme par exemple les anti-sens [comme GEM92, GPI-2A...], parmi les inhibiteurs de la famille des modulateurs de dNTP cellulaire [comme hydroxyurée], parmi les inhibiteurs de cytokines [comme TNF], parmi les inhibiteurs d'entrée des VIHs [comme T20, SPC-3...], ainsi que parmi les agents constituants les classes therapeutiques utilisées dans les approches vaccinales tant par biotechnologie [comme HIVAC-1e, ALVAC...] que par composés agissant sur la réponse immune [comme RG-8394].

Notamment le dérivé de cyclosporine selon l'invention manifeste un effet de synergie particulièrement intéressant lorsqu'il est associé avec l'AZT, le ddI et le Sasquinavir.

Les compositions pharmaceutiques comprenant de telles associations, éventuellement en présence d'excipients pharmaceutiquement acceptables, entrent également dans le cadre de la présente invention.

L'exemple suivant illustre une composition selon l'invention.

### Exemple

On prépare une formulation administrable par voie orale et ayant la composition suivante :

| | |
|---|---|
| [(R)-méthylthio-Sar]³-[4'-hydroxy-MeLeu]⁴-cyclosporine A | 250 mg |
| Stéarate de magnésium | 3 mg |
| Acidsol | 15 mg |
| Silice colloidale | 2 mg |
| Lactose | 130 mg |

## Revendications

1. Un dérivé de la cyclosporine **caractérisé en ce qu'**il répond à la formule :

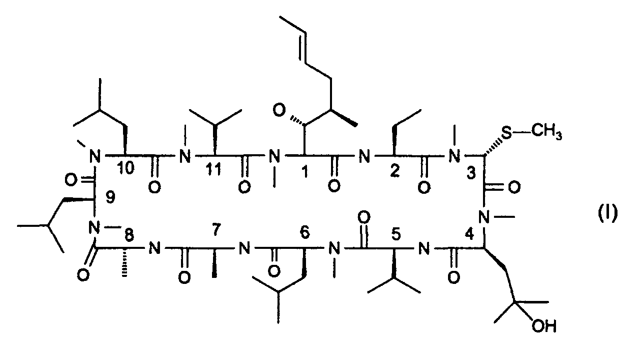

2. Procédé de préparation d'un dérivé de cyclosporine selon la revendication 1, **caractérisé en ce que** l'on fait agir le disulfure de diméthyle, sur une forme activée d'un dérivé [4'-hydroxy-MeLeu]⁴ cyclosporine de formule :

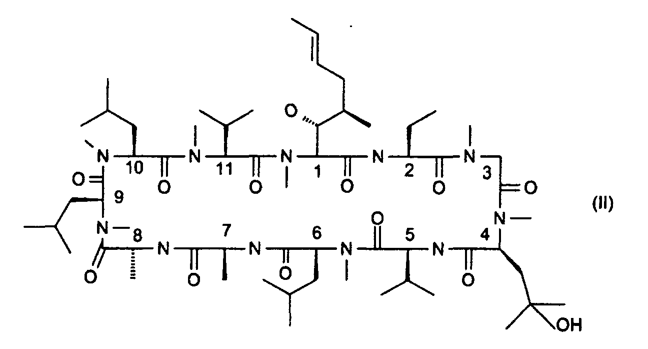

3. Composition pharmaceutique **caractérisée en ce qu'**elle contient le produit selon la revendication 1, à l'état pur ou en association avec tout diluant ou adjuvant compatible et pharmaceutiquement acceptable et/ou en association avec un autre principe actif antiviral, immunomodulateur ou antimicrobien.

4. Associations synergisantes **caractérisées en ce qu'**elles comprennent le dérivé de cyclosporine selon la revendications 1, et au moins un autre agent connu pour son activité anti-rétrovirus.

## Patentansprüche

1. Cyclosporin-Derivat, **dadurch gekennzeichnet, daß** es der allgemeinen Formel (I) 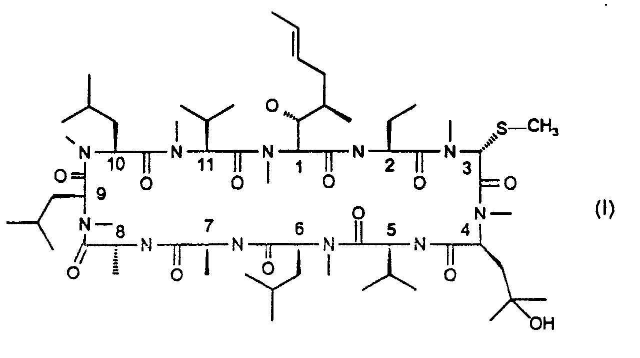 entspricht.

2. Verfahren zur Herstellung eines Cyclosporin-Derivates nach Anspruch 1, **dadurch gekennzeichnet, daß** man Dimethyldisulfid auf eine aktivierte Form eines [4'-Hydroxy-MeLeu]⁴-cyclosporin-Derivates der Formel (II) 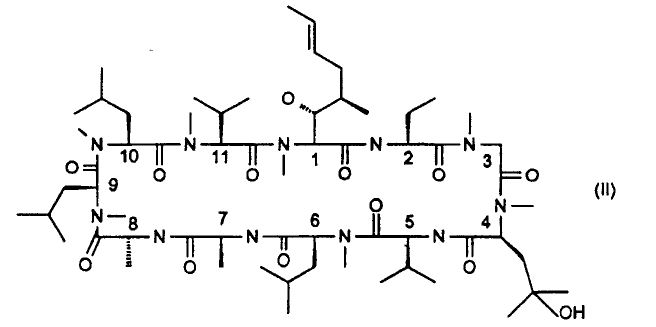 einwirken läßt.

3. Pharmazeutische Zusammensetzung, **dadurch gekennzeichnet, daß** sie das Produkt nach Anspruch 1 in reinem Zustand oder in Assoziation mit jedem kompatiblen und pharmazeutisch akzeptablen Verdünnungsmittel oder Zusatzstoff und/oder in Assoziation mit einem anderen antiviralen, immunomodulierenden oder antimikrobiellen Wirkstoff enthält.

4. Synergistische Assoziationen, **dadurch gekennzeichnet, daß** sie das Cyclosporin-Derivat nach Anspruch 1 und mindestens ein anderes Mittel umfassen, das für seine Anti-Retrovirus-Aktivität bekannt ist.

## Claims

1. Cyclosporin derivative, **characterized in that** it corresponds to the formula :

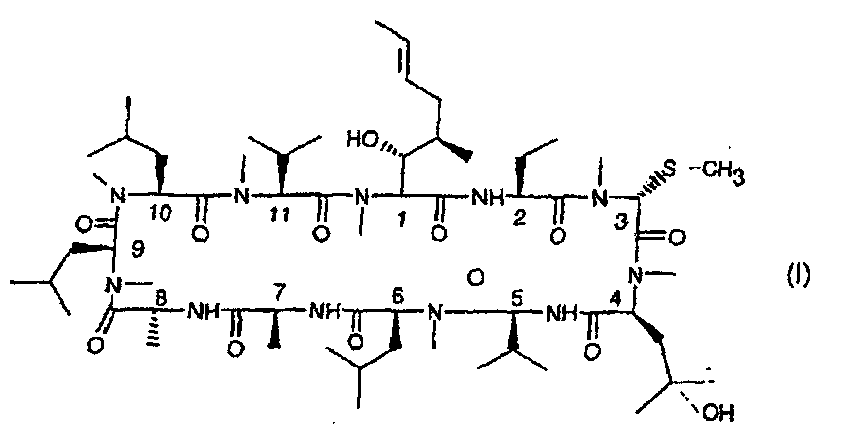

2. Process for the preparation of a cyclosporin derivative according to Claim 1, **characterized in that** dimethyl disulphide is reacted with an activated form of a [4'-hydroxy-MeLeu]⁴-cyclosporin derivative of formula:

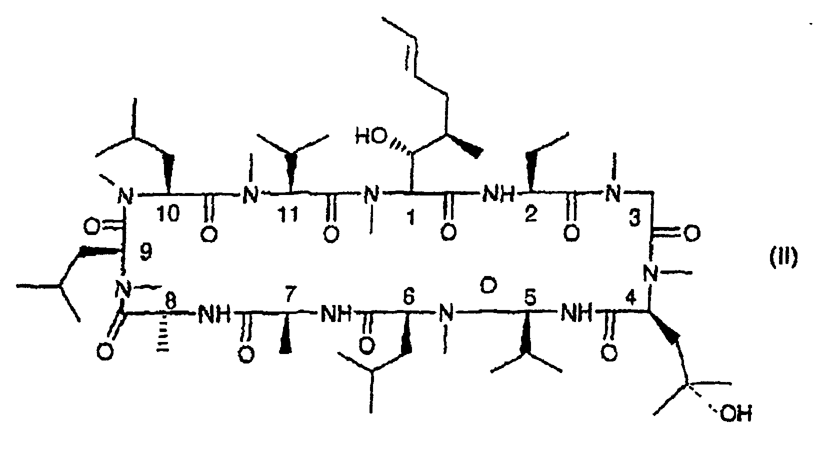

3. Pharmaceutical composition, **characterized in that** it contains the product according to Claim 1, in the pure state or in combination with any compatible and pharmaceutically acceptable diluent or adjuvant and/or in combination with another antiviral, immunomodulating or antimicrobial active principle.

4. Synergizing combinations, **characterized in that** they comprise the cyclosporin derivative according to Claim 1 and at least one other agent known for its anti-retrovirus activity.
